# EUROPEAN PATENT APPLICATION

(11) **EP 4 563 696 A1**
(43) Date of publication of application: **04.06.2025**
(21) Application number: 24215508.3
(22) Date of filing: 26.11.2024
(51) Int. Cl.: C12N 1/14, A61K 36/06

(54) **METHOD OF PREPARATION OF BIOMASS TO PROMOTE HEALING OF HYPERTROPHIC SCARS AND KELOIDS, BIOMASS AND ITS USE**

(30) Priority: 01.12.2023 SK 1522023
(71) Applicant: Slavka, Jancova, Sliac (SK)
(72) Inventor: Jancová, Slávka, Sliac (SK)
(74) Representative: Wörz, Volker Alfred

(57) **Abstract**

The method of preparation of biomass to support healing of hypertrophic scars and keloids, contains the steps:
Culturing of mycelium of the filamentous fungus,
Preparation of a pure culture of the filamentous fungus,
Verification of the generic and species affiliation of the filamentous fungus,
and Finalization of the biomass.

The biomass produced in this way is deposited on a nanotechnology substrate in the form of a foil. Biomass according to this solution is suitable for supporting the healing of hypertrophic scars and keloids or to test medicinal substances intended for the skin.

## Description

### Field of technology

The invention relates to a method of preparing a biomass intended to support healing of hypertrophic scars and keloids, the biomass produced in this way and its use.

### Prior art

In humans and other mammals, wound injury triggers an organized complex cascade of cellular and biochemical events that, in most cases, leads to wound healing. An ideally healed wound is one that restores normal anatomic structure, function, and appearance at the cellular, tissue, organ, and organismal levels.

Wound healing, whether initiated by trauma, microbes, or foreign materials, proceeds by a process involving a number of overlapping phases, including inflammation, epithelialization, angiogenesis, and matrix deposition. Normally, these processes lead to a mature wound and a certain degree of scar formation. Although inflammation and reparation proceed mostly in a prescribed manner, the sensitivity of the process depends on the balance of various wound-healing molecules, for example, from a network of regulatory cytokines and growth factors.

Wounds that do not heal in the normal or expected way, including chronic wounds such as diabetic foot ulcers, decubitus ulcers, and venous leg ulcers (VLUs), are a growing problem worldwide. For example, it is estimated that 1-2% of the population in Western countries will encounter a chronic wound during their lifetime. Chronic wounds represent a major economic burden on health services.

Various therapies for keloids have so far had only limited success. Existing efforts to manage hypertrophic scars and keloids include surgical intervention, mechanical pressure, steroids, X-ray irradiation and cryotherapy. Disadvantages are said to be associated with each of these methods. For example, surgical removal of scar tissue can often be incomplete and can lead to the development of hypertrophic scars and keloids at the incision and suture sites, i.e. scars often recur after surgical removal of the keloid, and steroid treatment may be performed. The results of such a procedure are unpredictable and often result in depigmentation of the skin. A simple surgical excision of keloid scars has a 50%-80% risk of recurrence.

A typical treatment has been a combination of surgical intervention with corticosteroid injection into the lesion or with radiotherapy. However, intralesional corticosteroid injection is prone to complications (fat atrophy, skin thinning, and pigment changes).

Adhesion formation is the process by which body tissues, which are usually separated, fuse with scar tissue. Adhesions are most often the result of surgical incision, abrasion or trauma. Adhesions can be formed after most surgical procedures, but occur with the highest frequency after general abdominal, gynecological, orthopedic, and cardiac surgeries. Although adhesions occur mostly after surgery, adhesions can also occur from tissue damage other than surgery, including traumatic injury, inflammatory disease, intraperitoneal chemotherapy, and radiotherapy.

Besides other complications, the presence of surgical adhesions may be associated with pain, discomfort and, for example, female infertility as a result of gynecological surgeries. For example, intestinal obstructions are a complication that is a result of surgical adhesions.

Vascular endothelial growth factors (VEGFs) are key regulators of angiogenesis during normal physiological and disease processes such as wound healing (Carmeliet and Jain, Nature 473, 298-307, 2011, Ferrara, Endocr Rev 25, 581-611, 2004, McColl et al., Apmis 112, 463-80, 2004). The VEGF family, which includes VEGF-A, VEGF-B, VEGF-C, VEGF-D and placental growth factor (PIGF), interacts with the high-affinity VEGF receptors (VEGF), VEGFR-1, VEGFR-2 and VEGFR-3 (Koch et al., Biochem J 437, 169-83, 2011; Olsson et al., Nat Rev Mol Cell Biol, 7, 359-71, 2006). VEGF-A binds to both VEGFR-1 and VEGFR-2, whereas PIGF and VEGF-B bind exclusively to VEGFR-1. VEGF-C and VEGF-D interact with both VEGFR-2 and VEGFR-3. VEGF also binds neuropilin coreceptors (NRP) -1 and NRP-2, which increase binding to VEGFR (Soker et al., J Cell Biochem 85, 357-68, 2002; Vadasz et al., Autoimmun Rev. 9, 825-829, 2010).

VEGF-A has been shown to promote angiogenesis by stimulation of endothelial cell proliferation, migration and survival and by support of vascular permeability, in particular via VEGFR-2 (Holmes et al., Cell Signal 19, 2003-2012, 2007). However, VEGFR-1 appears to play a role in endothelial cell differentiation and migration, possibly by acting as a ligand-binding molecule and sequestering VEGF-A from VEGFR-2 signaling (Shibuya, Angiogenesis 9, 225-230, 2006). During skin tissue repair, VEGF-A is highly expressed by keratinocytes and stimulates the formation of new blood vessels in the wound bed, thus supplying nutrients and oxygen necessary for skin regeneration (Barrientos et al., Wound Repair Regen 16, 585-601, 2008; Brown et al., J Exp Med 176, 1375-1379, 1992; Nissen et al., Am J Pathol 152, 1445-1452, 1998).

Document US 6753454 describes a new fiber comprising a substantially homogeneous blend of a hydrophilic polymer and a polymer that is at least slightly hydrophobic. The fiber optionally contains a pH adjusting compound. The method of fiber production comprises electrostatic fibrillation of the fibers of a substantially homogeneous polymer solution. A method of treating a wound or other area of a patient requiring protection from contamination includes electrostatic fibrillation of a substantially homogeneous polymer solution to form a bandage. An apparatus for electrostatic fibrillation a bandage is described.

Document US 7794219 describes an electrostatic fibrillation device for generating a coating from a liquefied polymer. The device contains: (a) a dispenser for dispensing the liquefied polymer; (b) a cavity having a longitudinal axis containing a first system of electrodes, the dispenser and the first system of electrodes being constructed and designed to dispense the liquefied polymer from the dispenser to form a number of polymer fibers moving along the longitudinal axis; and (c) a mechanism for displacing the polymer fibers from the cavity in a direction toward an object to form a coating on the object.

Document Shi-Cong Xu et al (Nanoscale, 2015, 7, 12351) describes an apparatus for electrostatic fibrillation (BOEA) based on miniaturization and integration. The device is separated from a conventional strong power supply, has tight integration of functional parts, and can be operated with one hand due to its small volume (10.5 × 5 × 3 cm³) and light weight (about 120 g). Various polymers, such as polyvinylpyrrolidone (PVP), polycaprolactone (PCL), polystyrene (PS), polylactic acid (PLA) and polyvinylidene fluoride (PVDF), have been successfully fibrillated electrolytically into fibers, which is confirmed by stable performance and good real quality - the ability of time control of the apparatus. These results show that BOEA could potentially be applied in many fields, especially in biomedical fields such as skin damage, wound healing, rapid hemostasis, etc.

In solving the issue of healing hypertrophic scars and keloids, ultrafine dry powders, also known as micro- and nano-powders, are the subject of increasing interest for pharmaceutical production. The advantage is that they provide a solution to many of the shortcomings of mixed drugs.

The active ingredients of the drug are manufactured, packaged and administered to the patient as pure dry powders without mixing them with solvents or other agents. The elimination of mixing steps simplifies the manufacturing process, reduces development and production costs, refines dosing, and extends the shelf life of the drug.

Many companies, such as Delsys Pharmaceutical Corp. of Princeton, New Jersey, have developed pharmaceutical manufacturing processes using electrostatic forces to deposit fine powders on a substrate. Electrostatic coating is the same principle as used in copying machines with dry toner. This technique is used to prepare and encapsulate dry powders into pills for oral administration.

The disadvantage of dry powders is that they are difficult to handle, tend to clump and stick in storage, and disperse if disturbed by even the slightest movement of air. These handling problems must be overcome if dry powder drugs are to be used effectively and safely, and special methods must be used to accurately process and administer the dose.

### The present invention

Despite progress in understanding the principles underlying the wound healing process, there remains an unmet need for appropriate wound care and tissue repair options and to improve and/or support wound healing, including for wounds that do not heal at the expected rate, such as wounds with delayed healing.

Incompletely healing wounds and impaired wound healing, such as those occurring in chronic wounds, scarring and abnormal or excessive scars, including keloid and hypertrophic scars, give rise to extensive scarring and scar contractures, as well as adhesions, including surgical adhesions.

Therefore, it is necessary to develop improved methods to address patient conditions that are caused by acute and chronic wounds, inflammation, fibrosis, scarring, and adhesions.

The essence of the invention lies in a method of preparation of biomass to support the healing of hypertrophic scars and keloids, which contains the following steps:
1. Culturing of mycelium of the filamentous fungus,
2. Preparation of a pure culture of the filamentous fungus,
3. Verification of the generic and species affiliation of the filamentous fungus, and
4. Finalization of the biomass.

When finalizing the biomass, a spore inoculum is prepared from a settled culture of filamentous fungus grown on PDA growth medium in 0.01% Tween water, then the culture is flooded over the entire surface with Tween water and the conidia are mechanically released. The mixture of mycelia and conidia is subsequently filtered through sterile gauze. The number of conidia 10⁸/ml is then determined in a Bürker chamber and the conidia are subsequently used to inoculate the growth medium in at least one culture vessel. After 48 hours of culturing on a 250 rpm shaker at 27 °C, the mycelium is separated from the growth medium by filtration under reduced pressure. Subsequently, the mycelium is captured on filter paper, and then the obtained biomass is dried at 50 °C for 1 hour. The dried biomass is crushed to a fine powder, which is placed in a freezer at a temperature of -20 °C for storage.

Culturing of the mycelium of the filamentous fungus is performed in such a way that, before inoculation, the growth media are sterilized in an autoclave at 120 °C and at 120 kPa for 20 minutes, and after the culturing of the mycelium of the filamentous fungus, the mycelium is transferred from the stock mixture to inoculate 100 ml of SGB. Subsequently, the mycelium is cultured dynamically for 7 days on a shaker 250 rpm at 27 °C.

The preparation of a pure culture of the filamentous fungus is performed in such a way that the cultured mycelium is transferred to solidified SGA and PDA growth media, where it is incubated for 3 days, subsequently for 5 days identical colonies of filamentous fungi are formed and conidia are formed on the colonies, which are subsequently transferred to fresh PDA and SGA growth media.

The genus and species affiliation of the filamentous fungus is preferably verified after 5 days of culturing, microscopically.

As a filter medium to trap mycelium, blue paper filters can preferably be used.

The obtained biomass is crushed into a fine powder, for example by application of liquid nitrogen.

The biomass produced in this way is subsequently spread on a nanotechnological substrate in the form of a film.

The biomass treated in this way can be used to support healing of hypertrophic scars and keloids. The use of biomass produced according to the present method has considerable advantages, both for patients and for medical personnel.

Advantages for patients:
- Ability to shower as early as 24 hours after application of the biomass
- Reduction of the need for frequent changes of clothing
- Elimination of wound friction resulting from bandages
- No bulky bandages that restrict movement; excellent movement abilities of the patient in affected areas such as joints
- Increased personal independence of the patient
- Reduced pain and shorter wound healing time
- Exponential growth in health-related quality of life (HRQL)

Advantages for medical personnel:
- Use of biomass prevents cross-contamination
- Reduces burden on caregivers
- Reduces patient treatment time
- Easy and quick dressing of difficult to dress areas
- Allows easy monitoring of wounds during healing
- Biomass is easily peeled off once the wound has healed.

### Overview of figures of drawings

Fig. 1a - Mycelium under the microscope
Fig. 1b - Treatment of a polycaprolactone fibrillated membrane by supplying a mycelium sample
Fig. 2 - PCL membrane - without treatment, under the microscope
Fig. 3 - Treatment of coarse mycelium under the microscope
Fig. 4 - Treatment of fine mycelium under the microscope

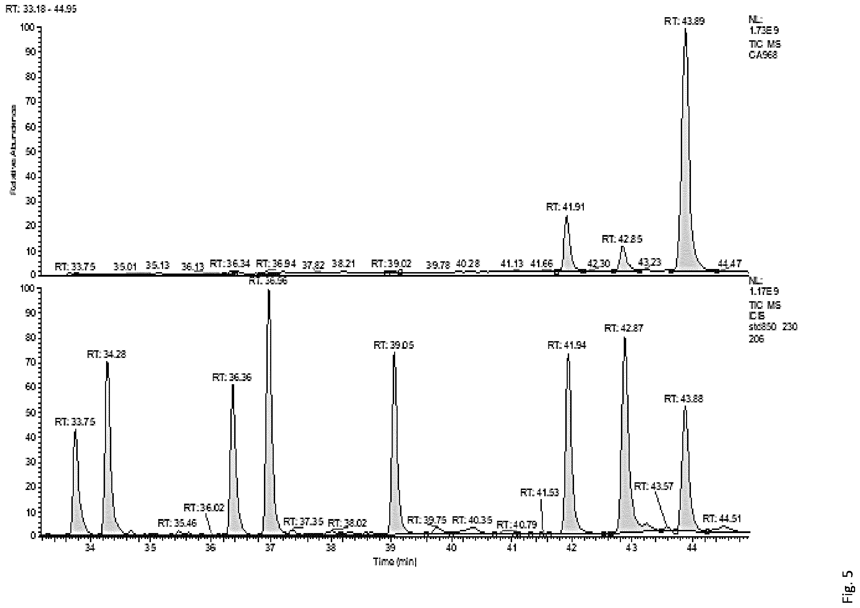

Fig. 5 Sugar analysis of mycelium of Penicillium chrysogenum, CA 968 parallel 1, H₂SO₄, mycelium sample of Penicillium chrysogenum above, standards below

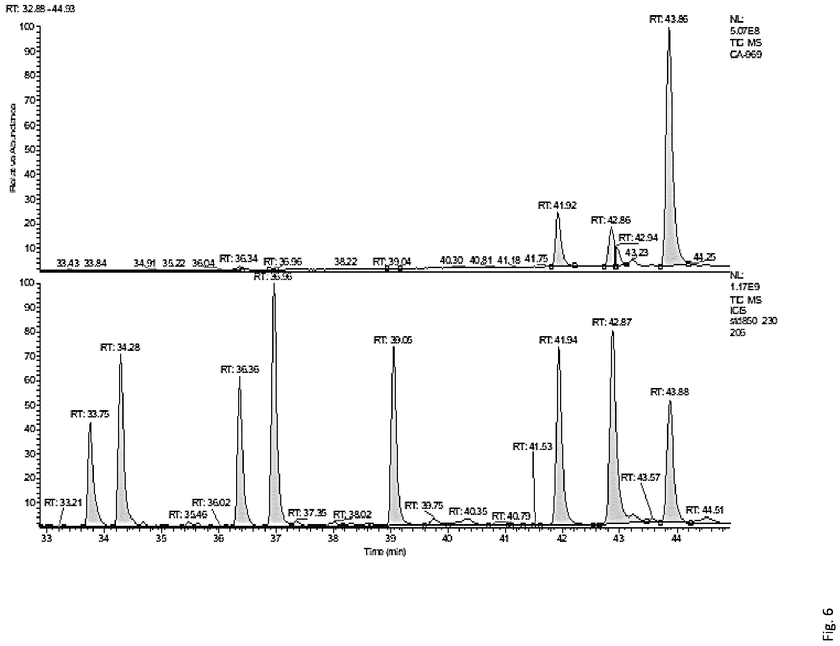

Fig. 6 Sugar analysis of mycelium of Penicillium chrysogenum, CA 969 parallel 2, H₂SO₄, mycelium sample of Penicillium chrysogenum above, standards below

### Examples

### Example 1

The preparation of biomass contains the following steps:
1. Culturing of mycelium of the filamentous fungus
2. Preparation of a pure culture of the filamentous fungus
3. Verification of the generic and species affiliation of the filamentous fungus
4. Finalization of the biomass.

The procedure is as follows:
Culture media are prepared, the growth media are sterilized in an autoclave at 120 °C and at 120 kPa for 20 minutes prior to inoculation.

After the culturing of the mycelium of the filamentous fungus, a part of the mycelium is transferred from the stock mixture to inoculate 100 ml of SGB. Mycelium is cultured for 7 days at 27 °C, dynamically on a shaker (250 rpm). On the seventh day, a part of the cultured mycelium is transferred to solidified SGA and PDA growth media.

After 3 days of incubation on solidified growth media, mycelium growth on PDA growth medium is recorded.

After 5 days, identical colonies of filamentous fungi are formed, and on the 6^{th} day, conidia (asexual spores) form on the colonies.

The originated conidia are transferred by microbial hook to fresh PDA and SGA growth media. Mycelium growth was observed already the next day. After 5 days of culturing, the genus affiliation of the filamentous fungus was microscopically verified.

From the sporulated 7-day-old culture grown on PDA growth medium, spore inoculum was prepared into 0.01% Tween water (0.01 g Tween 80 in 100 ml distilled water).

The culture was flooded over the entire surface with Tween water, the conidia were released with a microbial hook, and the mixture of mycelia and conidia was filtered through 3 times folded sterile gauze to reduce the number of mycelium fragments. Most of the mycelium was captured on the sterile gauze and mainly Penicillum chrysogennum conidia made it into the filtrate.

The number of conidia was determined to be 10⁸/ml in the Bürker chamber.

500 µl of conidia were used to inoculate 500 ml of growth medium in a 1-liter culture vessel. 4 culture vessels were inoculated simultaneously.

Biomass was obtained after 48 hours of culturing on a shaker 250 rpm, at 27 °C. After 48 hours, the mycelium separated from the growth medium by filtration under reduced pressure (vacuum filtration). The mycelium was captured on filter paper of the type Filtrak No. 390 (blue paper filters). The biomass was dried in a dryer at 50 °C for 1 hour, and the mycelium was crushed to a fine powder using liquid nitrogen. The biomass was placed in a freezer, where it was stored at a temperature of -20 °C, and in this state, the biomass is ready to be transferred for further use.

### Detailed description of the steps

### 1^{st} step: Culturing of mycelium of the filamentous fungus

Culture media that were used:
Potato glucose agar (PDA): 0.4 g potato extract, glucose, 2 g agar, 100 ml distilled water, pH of growth medium adjusted to a value of 6.4-6.6.
Sabouraud's broth (SGB): 1g peptone, 2 g glucose, 100 ml distilled water, pH of growth medium adjusted to a value of 6,4-6,6
Sabouraud's agar (SGA): 1g peptone, 2 g glucose, 2 g agar, 100 ml distilled water, pH of growth medium adjusted to a value of 6.4-6.6

Growth media were sterilized in an autoclave at 120 °C, 120 kPa for 20 minutes before inoculation.

Treatment of polycaprolactone fibrillated membrane by supplying a mycelium sample was analyzed by scanning electron microscopy (SEM) and X-ray photoelectron spectroscopy (XPS). Selected results are shown in Figures.

The following types of mycelium samples were used:
- Mycelium (coarse and fine)
- PCL nanofiber membrane
- PCL nanofiber membrane after treatment 1, 2, 3
- PCL nanofiber membrane after treatment 1, 2, 3 and mycelium attachment
- PCL nanofiber membrane after treatment 3, mycelium attachment and fixations

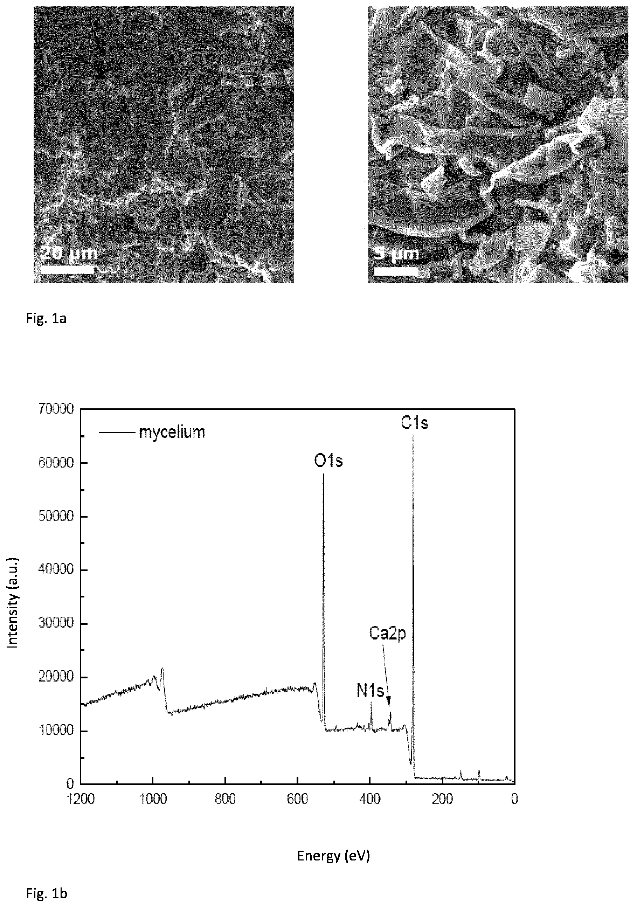

**Table 1, description to Figures 1a and 1b:**

| **Element** | **Abundance* (at. 96)** |
|---|---|
| carbon | 73.5±0.3 |
| oxygen | 20.8±0.2 |
| nitrogen** | 3.0-6.7 |
| calcium** | 0.3-0.8 |
| sulfur** | 0.1-0.3 |
| phosphorus** | 0.1-0.3 |
| sodium** | 0.1-0.3 |

| | |
|---|---|
| * Atomic abundance of individual elements determined from highly resolved XPS spectra. Also valid for all following elemental compositions. ** Atomic abundance of trace elements varied considerably within the mycelium sample. | |

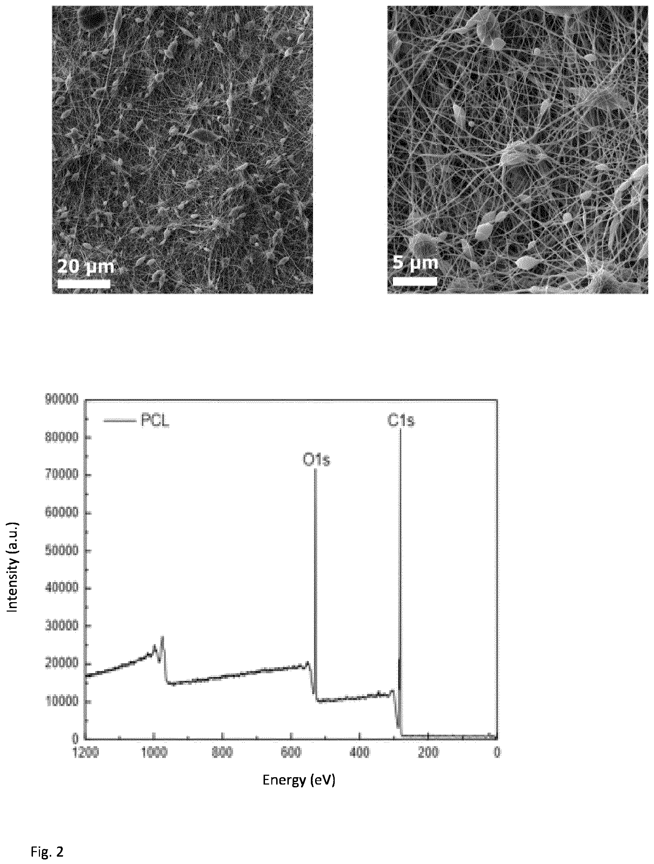

**Table 2, description to Figure 2:**

| **Element** | **Abundance* (at. %)** |
|---|---|
| carbon | 78 |
| oxygen | 22 |
| nitrogen | - |
| calcium | - |
| sulfur | - |
| phosphorus | - |
| sodium | - |

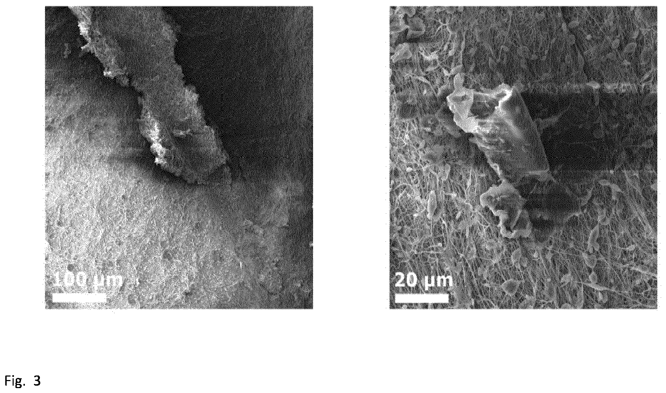

In Fig. 3 the mycelium is coarse; for this sample, the mycelium failed to establish homogeneously on the surface of the entire membrane after any of the treatments tested. Large pieces of mycelium were captured only sporadically for all methods.

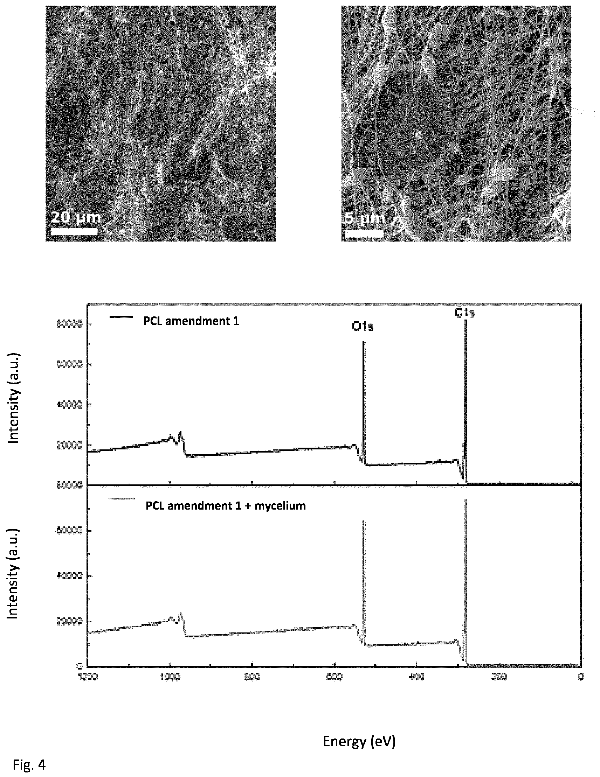

**Table 3, description to Figure 4:**

| **Element** | **Treatment 1 (at. %)** | **Treatment 1** + **mycelium (at. %)** |
|---|---|---|
| carbon | 78.0 | 77.2 |
| oxygen | 22.0 | 22.0 |
| nitrogen | - | 0.6 |
| calcium | - | 0.07 |
| sulfur | - | - |
| phosphorus | - | 0.16 |
| sodium | - | - |

Based on the results obtained from SEM and XPS, finely ground mycelium and membrane with treatment 2, treatment 3 and treatment 3 with fixation were used to prepare the final samples.

### 2^{nd} step: Preparation of a pure culture of the filamentous fungus - revival of filamentous fungus

After culturing of the mycelium of the filamentous fungus, a portion of the mycelium was transferred from the stock mixture to the solidified PDA, and SGA of the culture medium to the center of the prepared agar plate. The mycelium did not grow. The material obtained for inoculation was then analyzed microscopically. The mycelium was broken, extensively vacuolized, indicating low mycelium viability. The mycelium phenotypically resembled a culture in the late growth phase, in the dieback phase.

Since the mycelium did not grow on solidified growth medium, we attempted to revive it in liquid SGB. Again, a part of the culture was taken for inoculation of 100 ml SGB. Mycelium was cultured for 7 days at 27 °C, dynamically on a shaker (250 rpm). On the 6^{th} day of culturing, the emergence of new mycelium was observed, from which an aliquot part was transferred to solidified SGA and PDA media on the 7^{th} day.

After 3 days of incubation on solidified growth media, mycelium growth was recorded on PDA growth medium, after 5 days 3 identical colonies of filamentous fungi started to form and on the 6^{th} day conidia (asexual spores) were forming on the colonies.

### 3^{rd} step: Verification of the generic and species affiliation of the filamentous fungus

The originated conidia were transferred by microbial hook to fresh PDA and SGA growth media. Mycelium growth was observed already the next day. After 5 days of culturing, the genus affiliation of the filamentous fungus was microscopically verified.

Macro appearance as well as microscopic observation confirmed the typical morphological features for the genus Penicillium sp. For detailed diagnosis, a method based on the ribosomal protein profile by mass spectroscopy was used as follows:
Conidia (10⁵) of filamentous fungus were used to inoculate liquid growth medium SGB in a 5 ml tube. Culturing was performed at 27 °C for 24 hours, dynamically on a shaker (250 rpm). Young mycelium was removed from the growth medium by centrifugation at (13,000 × g), washed 3 times with distilled water, and subsequently the ribosomal proteins were extracted in an ethanol-water mixture (300 µl : 900 µl) on a vortex with vigorous stirring for 2 minutes. Centrifugation (13,000 × g) followed, the supernatant was removed, and the sediment was extracted in a mixture of 70% formic acid and acetomitrile (1 : 1).

Centrifugation (13,000 × g) then followed, the supernatant was removed, which was analyzed by mass spectrometry using the biotyping method. The originated mass profile of ribosomal proteins was compared with that in databases (Bruker - Daltonics Fungal library). The filamentous fungus was identified to species level with an excellent identity score of 2.2 as Penicillium chrysogenum.

### 4^{th} step: Finalization of the biomass

From the sporulated 7-day-old culture grown on PDA growth medium, spore inoculum was prepared into 0.01% Tween water (0.01g Tween 80 in 100 ml distilled water).

The culture was flooded over the entire surface with Tween water, the conidia were released with a microbial hook, and the mixture of mycelia and conidia was filtered through 3 times folded sterile gauze to reduce the number of mycelium fragments. Most of the mycelium was captured on the sterile gauze and mainly Penicillum chrysogennum conidia made it into the filtrate.

The number of conidia was determined to be 10⁸/ml in the Bürker chamber.

500 µl of conidia were used to inoculate 500 ml of growth medium in a 1-liter culture vessel. 4 culture vessels were inoculated simultaneously.

Biomass was obtained after 48 hours of culturing on a shaker 250 rpm, at 27 °C. After 48 hours, the mycelium separated from the growth medium by filtration under reduced pressure (vacuum filtration). The mycelium was captured on filter paper of the type Filtrak No. 390 (blue paper filters). The biomass was dried in a dryer at 50 °C for 1 hour, and the mycelium was crushed to a fine powder using liquid nitrogen. The biomass was placed in a freezer, where it was stored at a temperature of -20 °C. In this state, the biomass is ready to be transferred for further use

The preparation of a pure culture of the filamentous fungus was performed in such a way that the cultured mycelium was transferred to solidified SGA and PDA growth media, where it was incubated for 3 days, subsequently for 5 days identical colonies of filamentous fungi were formed and conidia were formed on the colonies, which were subsequently transferred to fresh PDA and SGA growth media.

The genus and species affiliation of the filamentous fungus was verified after 5 days of culturing, microscopically.

As a filter medium to trap mycelium, blue paper filters were used.

The obtained biomass was crushed into a fine powder by application of liquid nitrogen.

The biomass applied to the film was used to fully cover the wound in the form of abraded skin, without massive bleeding. The injured person felt numbness of the wound within a few minutes. After 2 hours from applying the film with biomass, the wound was still calm, without signs of inflammation, and numb. After 24 hours from applying the film with biomass, the film was removed from the wound. The wound was sealed, the formed scar was free of inflammation and it was subsequently healing without any problems.

### Example 2

The biomass was produced in the same way as in Example 1. The produced sample had dimensions of 5 cm × 5 cm, the thickness of the sample is 144±4 µm, weight 100 mg. The biomass sample was subjected to tests for bacterial gene mutations.

The tests were performed by the Výzkumný ústav organických syntéz a.s., testing device SLP - Centrum ekologie, toxikologie a analytiky, No. 296, 533 54 Rybitví, Czech Republic.

Number of study: 23V01162/12
Beginning and end of the experimental phase of the study: 24.3.2023 to 24.4.2023.

**Table 4 data provided by the study sponsor:**

| | | | | |
|---|---|---|---|---|
| Batch No.: | | 1 | | |
| Molecular weight: | | 45,000 | | |
| Composition from the tested material: | | | | |
| Main component: | | | CAS No.: | Concentration (wt. %): |
| Name: | | | | |
| Mycelium-Penicillium chrysogenum | | | Not stated | 99.9% |
| Impurities: | | | | |
| Name: | | | | |
| Chloroform, ethanol, acetic acid | | | Not stated | max. 0.1% |
| Release liner: | | | | |
| Polymer polyester, polycaprolactone | | | | |
| Appearance: | White nanofoil - carrier, solid form (the active substance is applied to the white nanofoil, the upper and lower part of the sample is blue - the deposit layer for the application of nanofibers, the white sample is tested). The produced sample is 5cm × 5cm, sample thickness is 144±4 µm, weight 100 mg. | | | |
| Sterilization: | | Yes, Ag | | |
| Nano material: | | Yes | | |
| Polymer material: | | No | | |
| Possible liquid residue: | | Not stated | | |
| Storage: | | Keep away from direct sunlight (dark film, closed box). | | |
| Expiry date: | | 2 years, 28.11.2024 | | |

Test site findings:

| | |
|---|---|
| Appearance: | White layer |
| Storage and handling during the study: | Store in the original packaging (plastic bag), in a dark and dry place, at room temperature. |

The preparation from the tested material was performed according to the standard STN EN ISO 10993-12. The mutagenicity testing was performed according to the standard STN EN ISO 10993-3 and OECD Test Guideline No. 471- Bacterial Reverse Mutation Test. Four Salmonella typhimurium indicator strains -TA 98, TA 100, TA 1535 and TA 1537 and one Escherichia coli WP2 uvrA strain were used for mutagenicity testing.

Method C according to the standard STN EN ISO 10993-3 was used for sample preparation. The tested material was cut and extracted in water (polar solvent) and in olive oil (non-polar solvent). For the experiments, the aqueous extract was diluted with water, the oil extract was diluted with dimethyl sulfoxide.

Spontaneous reversion, solvent controls and positive control were compared with historical controls in the laboratory.

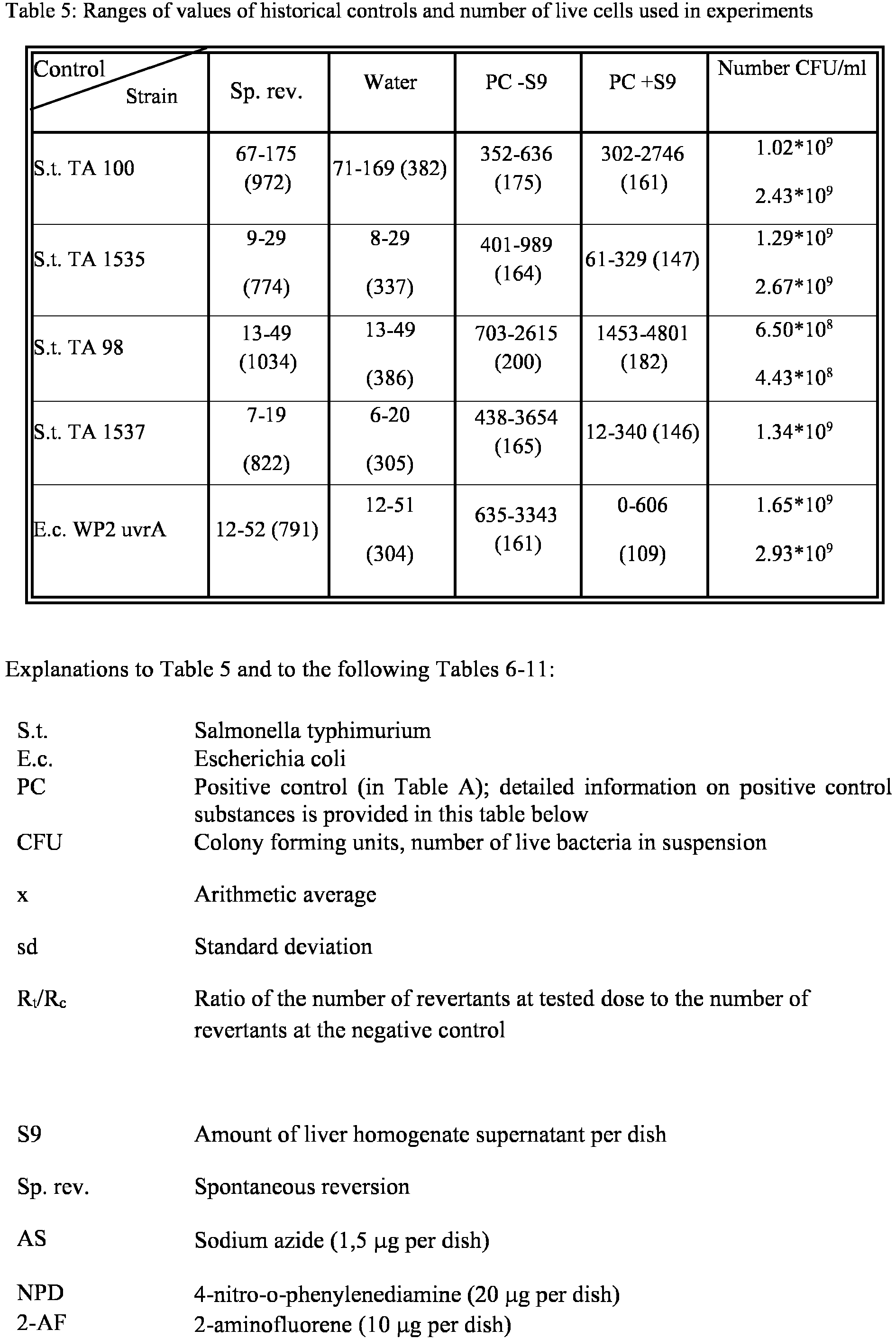

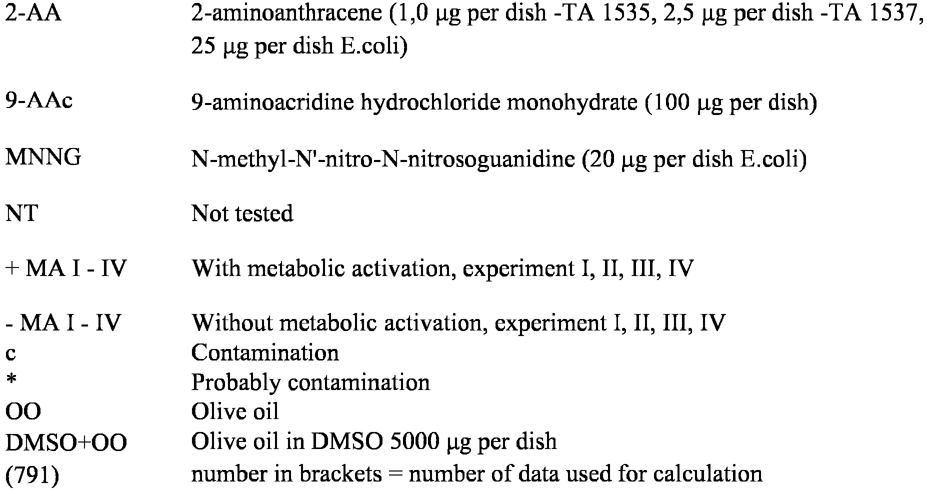

The first two mutagenicity experiments (with extracts in polar and non-polar vehicle) were performed by a standard dish test with concentrations of 50, 150, 500, 1500 and 5000 µg per dish applied in a volume of 0.1 ml. Experiments without metabolic activation and experiments with metabolic activation were performed using rat liver homogenate supernatant (30 µl S9 per dish) and a mixture of cofactors.

**Table 6: Effect of aqueous extract of the test material on the strain S. typhimurium TA 100**

| Cone. µg/dish | S9 µl | Number of revertants per dish | | | Average ±sd | Rₜ/R_{c} | S9 µl | Number of revertants per dish | | | Average ±sd | Rₜ/R_{c} |
|---|---|---|---|---|---|---|---|---|---|---|---|---|
| | | - MA I (31/03/2023) | | | | | | + MA (31/03/2023) | | | | |
| Sp. rev. | 0 | 132 | 170 | 153 | 152±16 | - | 30 | 134 | 127 | 132 | 131±3 | - |
| water | 0 | 135 | 137 | 157 | 143±10 | - | 30 | 150 | 131 | 130 | 137±9 | - |
| 50 | 0 | 135 | 136 | 140 | 137±2 | 1.0 | 30 | 133 | 151 | 153 | 146±9 | 1.1 |
| 150 | 0 | 132 | 141 | 145 | 139±5 | 1.0 | 30 | 150 | 143 | 169 | 154±11 | 1.1 |
| 500 | 0 | 132 | 155 | 113 | 133±17 | 0.9 | 30 | 130 | 144 | 132 | 135±6 | 1.0 |
| 1500 | 0 | 127 | 145 | 135 | 136±7 | 0.9 | 30 | 168 | 137 | 134 | 146±15 | 1.1 |
| 5000 | 0 | 126 | 139 | 108 | 124±13 | 0.9 | 30 | 140 | 145 | 142 | 142±2 | 1.0 |
| AS/2-AF | 0 | 488 | 622 | NT | 555±67 | 3.9 | 20 | 1200 | 1060 | NT | 1130±70 | 8.2 |

| Conc. % of leachate | | - MA III (17/04/2023) | | | | | | + MA III (17/04/2023) | | | | |
|---|---|---|---|---|---|---|---|---|---|---|---|---|
| Sp. rev. | 0 | 93 | 82 | 83 | 86±5 | - | 30 | 90 | 80 | c | 85±5 | - |
| water | 0 | 84 | 82 | 75 | 80±4 | - | 30 | 88 | 81 | 96 | 88±6 | - |
| 5 | 0 | 85 | 89 | 74 | 83±6 | 1.0 | 30 | 78 | 93 | 95 | 89±8 | 1.0 |
| 10 | 0 | 79 | 81 | 92 | 84±6 | 1.0 | 30 | 81 | 79 | 89 | 83±4 | 0.9 |
| 25 | 0 | 69 | 86 | 84 | 80±8 | 1.0 | 30 | 107 | 94 | 82 | 94±10 | 1.1 |
| 50 | 0 | 95 | 101 | 98 | 98±2 | 1.2 | 30 | 99 | 82 | 87 | 89±7 | 1.0 |
| 100 | 0 | 78 | 89 | 85 | 84±5 | 1.0 | 30 | 73 | 86 | 80 | 80±5 | 0.9 |
| AS/2-AF | 0 | 576 | 581 | NT | 579±3 | 7.2 | 20 | 1220 | 1100 | NT | 1160±60 | 13.1 |

**Table 7: The effect of the extract from the tested material in olive oil on the strain S. typhimurium TA 100**

| Cone. µg/dish | S9 µl | Number of revertants per dish | | | Average ±sd | Rₜ/R_{c} | S9 µl | Number of revertants per dish | | | Average ±sd | Rₜ/R_{c} |
|---|---|---|---|---|---|---|---|---|---|---|---|---|
| | | - MA II (06/04/2023) | | | | | | + MA II (06/04/2023) | | | | |
| Sp. rev. | 0 | 94 | 83 | 86 | 88±5 | - | 30 | 80 | 82 | 94 | 85±6 | - |
| DMSO+OO | 0 | 95 | 89 | 87 | 90±3 | - | 30 | 84 | 92 | 92 | 89±4 | - |
| 50 | 0 | 109 | 67 | 80 | 85±18 | 0.9 | 30 | 82 | 97 | 90 | 90±6 | 1.0 |
| 150 | 0 | 87 | 108 | 93 | 96±9 | 1.1 | 30 | 96 | 84 | 69 | 83±11 | 0.9 |
| 500 | 0 | 91 | 83 | 95 | 90±5 | 1.0 | 30 | 89 | 81 | 100 | 90±8 | 1.0 |
| 1500 | 0 | 91 | 98 | 103 | 97±5 | 1.1 | 30 | 84 | 84 | 87 | 85±1 | 1.0 |
| 5000 | 0 | 88 | 81 | 94 | 88±5 | 1.0 | 30 | 79 | 72 | 87 | 79±6 | 0.9 |
| AS/2-AF | 0 | 483 | 534 | NT | 509±26 | 5.6 | 20 | 1220 | 1350 | NT | 1285±65 | 14.4 |

| Conc. % of leachate | | - MA IV (24/04/2023) | | | | | | + MA IV (24/04/2023) | | | | |
|---|---|---|---|---|---|---|---|---|---|---|---|---|
| Sp. rev. | 0 | 83 | 81 | 88 | 84±3 | - | 30 | 91 | 86 | 82 | 86±4 | - |
| OO | 0 | 93 | 98 | 74 | 88±10 | - | 30 | 80 | 80 | 85 | 82±2 | - |
| 5 | 0 | 78 | 79 | 86 | 81±4 | 0.9 | 30 | 75 | 85 | 86 | 82±5 | 1.0 |
| 10 | 0 | 88 | 91 | 73 | 84±8 | 1.0 | 30 | 75 | 77 | 73 | 75±2 | 0.9 |
| 25 | 0 | 83 | 73 | 78 | 78±4 | 0.9 | 30 | 90 | 80 | 91 | 87±5 | 1.1 |
| 50 | 0 | 69 | 82 | 73 | 75±5 | 0.8 | 30 | 88 | 77 | 90 | 85±6 | 1.0 |
| 100 | 0 | 91 | 89 | 75 | 85±7 | 1.0 | 30 | 103 | 85 | 89 | 92±8 | 1.1 |
| AS/2-AF | 0 | 464 | 454 | NT | 459±5 | 52 | 20 | 1110 | 1030 | NT | 1070±40 | 13.1 |

No increase in the number of revertants was observed at any concentration for any indicator strain.

The second two experiments (again with extracts in polar and non-polar vehicle) were performed with increased concentrations of the leachates, namely 5, 10, 25, 50, 100% of extract (according to ISO/TR 10993-33). The other test conditions remained maintained.

**Table 8: The effect of the water extract from the tested material on the strain S. typhimurium TA 1535**

| Cone. µg/dish | S9 µl | Number of revertants per dish | | | Average ±sd | Rₜ/R_{c} | 59 µl | Number of revertants per | | | Average ±sd | Rₜ/R_{c} |
|---|---|---|---|---|---|---|---|---|---|---|---|---|
| | | - MA I (31/03/2023) | | | | | | + MA I (31/03/2023) | | | | |
| Sp. rev. | 0 | 14 | 19 | 10 | 14±4 | - | 30 | 18 | 14 | 17 | 16±2 | - |
| water | 0 | 10 | 17 | 14 | 14±3 | - | 30 | 15 | 11 | 14 | 13±2 | - |
| 50 | 0 | 14 | 15 | 23 | 17±4 | 1.3 | 30 | 15 | 14 | 15 | 15±0 | 1.1 |
| 150 | 0 | 14 | 23 | 21 | 19±4 | 1.4 | 30 | 15 | 10 | 18 | 14±3 | 1.1 |
| 500 | 0 | 12 | 12 | 16 | 13±2 | 1.0 | 30 | 14 | 18 | 26 | 19±5 | 1.5 |
| 1500 | 0 | 15 | 18 | 14 | 16±2 | 1.1 | 30 | 14 | 14 | 11 | 13±1 | 1.0 |
| 5000 | 0 | 13 | 9 | 15 | 12±2 | 0.9 | 30 | 13 | 16 | 13 | 14±1 | 1.1 |
| A5/2-AA | 0 | 812 | 821 | NT | 817±5 | 59.7 | 20 | 123 | 164 | NT | 144±21 | 10.8 |

| Conc. % of leachate | | - MA III (17/04/2023) | | | | | | + MA III (17/04/2023) | | | | |
|---|---|---|---|---|---|---|---|---|---|---|---|---|
| Sp. rev. | 0 | 17 | 18 | 11 | 15±3 | - | 30 | 13 | 13 | 12 | 13±0 | - |
| water | 0 | 13 | 12 | 15 | 13±1 | - | 30 | 13 | 16 | 18 | 16±2 | - |
| 5 | 0 | 17 | 17 | 18 | 17±0 | 1.3 | 30 | 13 | c | 13 | 13±0 | 0.8 |
| 10 | 0 | 17 | 16 | 10 | 14±3 | 1.1 | 30 | 18 | 11 | 16 | 15±3 | 1.0 |
| 25 | 0 | 9 | 18 | 11 | 13±4 | 1.0 | 30 | 12 | 11 | 20 | 14±4 | 0.9 |
| 50 | 0 | 17 | 10 | 9 | 12±4 | 0.9 | 30 | 11 | 13 | 10 | 11±1 | 0.7 |
| 100 | 0 | 15 | 11 | c | 13±2 | 1.0 | 30 | 18 | 15 | 12 | 15±2 | 1.0 |
| A5/2-AA | 0 | 870 | 876 | NT | 873±3 | 65.5 | 20 | 133 | 129 | NT | 131±2 | 8.4 |

**Table 9: The effect of the extract from the tested material in olive oil on the strain S. typhimurium TA 1535**

| Conc. µg/dish | S9 µl | Number of revertants per dish | | | Average ±sd | Rₜ/R_{c} | 59 µl | Number of revertants per dish | | | Average ±sd | Rₜ/R_{c} |
|---|---|---|---|---|---|---|---|---|---|---|---|---|
| | | - MA II (06/04/2023) | | | | | | + MA II (06/04/2023) | | | | |
| Sp. rev. | 0 | 10 | 17 | 8 | 12±4 | - | 30 | 11 | 9 | 13 | 11±2 | - |
| DMSO+OO | 0 | 12 | 15 | 10 | 12±2 | - | 30 | 8 | 9 | 11 | 9±1 | - |
| 50 | 0 | 9 | 10 | 15 | 11±3 | 0.9 | 30 | 13 | 19 | 13 | 15±3 | 1.6 |
| 150 | 0 | 15 | 11 | 11 | 12±2 | 1.0 | 30 | 8 | 10 | 17 | 12±4 | 1.3 |
| 500 | 0 | 14 | 9 | 5 | 9±4 | 0.8 | 30 | 13 | 14 | 16 | 14±1 | 1.5 |
| 1500 | 0 | 11 | 13 | 12 | 12±1 | 1.0 | 30 | 12 | 13 | 9 | 11±2 | 1.2 |
| 5000 | 0 | 11 | 11 | 58* | 27±22 | 2.2 | 30 | 11 | 14 | 8 | 11±2 | 1.2 |
| A5/2-AA | 0 | 831 | 840 | NT | 836±5 | 67.7 | 20 | 193 | 162 | NT | 178±16 | 19.0 |

| Conc. % of leachate | | - MA IV (24/04/2023) | | | | | | + MA IV (24/04/2023) | | | | |
|---|---|---|---|---|---|---|---|---|---|---|---|---|
| Sp. rev. | 0 | 12 | 17 | 11 | 13±3 | - | 30 | 18 | 18 | 13 | 16±2 | - |
| OO | 0 | 14 | 11 | 18 | 14±3 | - | 30 | 16 | 20 | 18 | 18±2 | - |
| 5 | 0 | 17 | 11 | 19 | 16±3 | 1.1 | 30 | 14 | 25 | 19 | 19±5 | 1.1 |
| 10 | 0 | 18 | 13 | 15 | 15±2 | 1.1 | 30 | 18 | 21 | 22 | 20+2 | 1.1 |
| 25 | 0 | 25 | 13 | 11 | 16±6 | 1.1 | 30 | 18 | 20 | 13 | 17±3 | 0.9 |
| 50 | 0 | 17 | 16 | 18 | 17±1 | 1.2 | 30 | 17 | 17 | 15 | 16±1 | 0.9 |
| 100 | 0 | 10 | 16 | 18 | 15±3 | 1.0 | 30 | 14 | 11 | 15 | 13±2 | 0.7 |
| A5/2-AA | 0 | 720 | 742 | NT | 731±11 | 51.0 | 20 | 97 | 116 | NT | 107±10 | 5.9 |

**Table 10: The effect of the water extract from the tested material on the strain S. typhimurium TA 9**

| Cone. µg/dish | S9 µl | Number of revertants per dish | | | Average ±sd | Rₜ/R_{c} | S9 µl | Number of revertants per dish | | | Average ±sd | Rₜ/R_{c} |
|---|---|---|---|---|---|---|---|---|---|---|---|---|
| | | - MA I (17/04/2023) | | | | | | + MA I (17/04/2023) | | | | |
| Sp. rev. | 0 | 37 | 47 | 43 | 42±4 | - | 30 | 39 | 45 | 37 | 40±3 | - |
| water | 0 | 31 | 41 | c | 36±5 | - | 30 | 39 | 43 | 41 | 41±2 | - |
| 50 | 0 | 35 | 40 | c | 38±3 | 1.0 | 30 | 49 | 40 | 49 | 46±4 | 1.1 |
| 150 | 0 | 41 | 35 | 33 | 36±3 | 1.0 | 30 | 37 | 40 | 37 | 38±1 | 0.9 |
| 500 | 0 | 39 | 30 | 51 | 40±9 | 1.1 | 30 | 39 | 48 | 41 | 43±4 | 1.0 |
| 1500 | 0 | 35 | 37 | 32 | 35±2 | 1.0 | 30 | 46 | 35 | 42 | 41±5 | 1.0 |
| 5000 | 0 | 36 | 31 | 40 | 36±4 | 1.0 | 30 | 42 | 36 | 41 | 40±3 | 1.0 |
| NPD/2-AF | 0 | 1140 | 1350 | NT | 1245±105 | 34.6 | 20 | 2570 | 2630 | NT | 2600±30 | 63.4 |

| Conc. % of leachate | | - MA III (21/04/2023) | | | | | | + MA III (21/04/2023) | | | | |
|---|---|---|---|---|---|---|---|---|---|---|---|---|
| Sp. rev. | 0 | 27 | 32 | 32 | 30±2 | - | 30 | 33 | 30 | 31 | 31±1 | - |
| water | 0 | 26 | 33 | 24 | 28±4 | - | 30 | 37 | 40 | 40 | 39±1 | - |
| 5 | 0 | 33 | 28 | 27 | 2913 | 1.1 | 30 | 39 | 40 | 40 | 40±0 | 1.0 |
| 10 | 0 | 29 | 29 | 33 | 3012 | 1.1 | 30 | 41 | 29 | 38 | 3615 | 0.9 |
| 25 | 0 | 43 | 31 | 23 | 32±8 | 1.2 | 30 | 27 | 36 | 30 | 31±4 | 0.8 |
| 50 | 0 | 37 | 36 | 30 | 34±3 | 1.2 | 30 | 35 | 26 | 24 | 28±5 | 0.7 |
| 100 | 0 | 31 | 28 | 24 | 28±3 | 1.0 | 30 | 40 | 34 | 31 | 35±4 | 0.9 |
| NPD/2-AF | 0 | 1150 | 1290 | NT | 1220±70 | 44.1 | 20 | 2480 | 2280 | NT | 2380±100 | 61.0 |

**Table 11: The effect of the extract from the tested material in olive oil on the strain S. typhimurium TA 98**

| Cone. µg/dish | S9 µl | Number of revertants per dish | | | Average ±sd | Rₜ/R_{c} | S9 µl | Number of revertants per dish | | | Average ±sd | Rₜ/R_{c} |
|---|---|---|---|---|---|---|---|---|---|---|---|---|
| | | - MA II (21/04/2023) | | | | | | + MA II (21/04/2023) | | | | |
| Sp. rev. | 0 | 27 | 32 | 32 | 30±2 | | 30 | 33 | 30 | 31 | 31±1 | |
| DMSO+OO | 0 | 25 | 29 | 25 | 26±2 | | 30 | 27 | 32 | 29 | 29±2 | |
| 50 | 0 | 25 | 26 | 41 | 31±7 | 1.2 | 30 | 28 | 24 | 35 | 29±5 | 1.0 |
| 150 | 0 | 29 | 27 | 24 | 27±2 | 1.0 | 30 | 25 | 30 | 30 | 28±2 | 1.0 |
| 500 | 0 | 25 | 17 | 25 | 22±4 | 0.8 | 30 | 28 | 24 | 33 | 28±4 | 1.0 |
| 1500 | 0 | 35 | 25 | 28 | 29±4 | 1.1 | 30 | 41 | 29 | 32 | 3415 | 1.2 |
| 5000 | 0 | 24 | 26 | 33 | 28±4 | 1.1 | 30 | 27 | 31 | 48 | 3519 | 1.2 |
| NPD/2-AF | 0 | 1150 | 1290 | NT | 1220±70 | 46.3 | 20 | 2480 | 2280 | NT | 2380±100 | 81.1 |

| Conc. % of leachate | | - MA IV (24/04/2023) | | | | | | + MA IV (24/04/2023) | | | | |
|---|---|---|---|---|---|---|---|---|---|---|---|---|
| Sp. rev. | 0 | 21 | 28 | 21 | 23±3 | | 30 | 33 | 39 | 30 | 34±4 | |
| OO | 0 | 28 | 28 | 21 | 26±3 | | 30 | 27 | 24 | 30 | 27±2 | |
| 5 | 0 | 28 | 18 | 28 | 25±5 | 1.0 | 30 | 37 | 30 | c | 34±4 | 1.2 |
| 10 | 0 | 26 | 23 | 29 | 26±2 | 1.0 | 30 | 28 | 30 | 20 | 26±4 | 1.0 |
| 25 | 0 | 22 | 27 | 25 | 25±2 | 1.0 | 30 | c | 26 | 26 | 26±0 | 1.0 |
| 50 | 0 | 25 | 26 | 25 | 25±0 | 1.0 | 30 | 32 | 26 | 23 | 27±4 | 1.0 |
| 100 | 0 | 26 | 20 | 33 | 26±5 | 1.0 | 30 | 31 | 26 | 31 | 29±2 | 1.1 |
| NPD/2-AF | 0 | 1260 | 1230 | NT | 1245±15 | 48.5 | 20 | 2860 | 2200 | NT | 2530±330 | 93.7 |

Simultaneously performed positive controls confirmed the sensitivity of the experiment to mutagens and the effectiveness of the metabolic activation system. Average numbers of colonies for the solvent control fell within the current range of historical controls in the laboratory.

Under the above arrangement, the tested material - biomass was non-mutagenic for all used indicator strains used, both without and with metabolic activation.

### Example 3

The biomass was produced in the same way as in Example 1.

The tests with the name of the study **"BIOKOŽA Senzibilizace kůže: Zkouška s vyšetšením lokalnich lymfatických uzlin"** (=BIOSKIN Skin sensitization: Test with examination of local lymph nodes) were performed by the Výzkumný ústav organických syntéz a.s., testing device SLP - Centrum ekologie, toxikologie a analytiky, No. 296, 533 54 Rybitví, Czech Republic.

Number of study: 23V02322/6
Beginning and end of the experimental phase of the study: 14.05. - 23. 05. 2023.

The goal of the LLNA test is to identify substances - sensitizers that cause the proliferation of lymphocytes in the lymph nodes that drain the place of application. This proliferation is a simple means of achieving an objective, quantitative measurement of sensitization.

The study is part of testing the biological compatibility of the tested material.

**Table 12: data provided by the study sponsor:**

| Batch 2 | | |
|---|---|---|
| **Composition from the tested material** | | |
| Main component: | | |
| Name: | CAS: | Concentration (wt. %): |
| **Mycelium-Penicillium chrysogenum** molecular weight 45,000, purity 99.9% | Not stated | Not stated |

| Impurity: | | |
|---|---|---|
| Name: | CAS: | Concentration (wt. %): |
| Chloroform, ethanol, acetic acid | Not stated | Max. 0.1 % |
| Releasable carrier: Polymer, polyester, polycaprolactone | | |
| **Appearance:** | White nanofoil - carrier, solid form (the active substance is applied to the white nanofoil, the upper and lower part of the sample is blue - the deposit layer for the application of nanofibers, the white sample is tested). The produced sample is 5cm × 5cm, sample thickness is 144±4 µm, weight 100 mg. | |
| **Sterilization:** | Yes (with help of Ag) | |
| **Nano material:** | Yes | |
| **Polymer material:** | No | |
| Possible liquid residues: | Not stated | |
| **Whether to use ultrasound for dissolution:** | Not stated | |
| **Storage conditions:** | Without contact with direct sunlight, in dark film, sealed | |
| **Expiry date:** | 2 years (15.3.2025) | |
| **Specific handling of the tested material:** | Not stated | |
| **Primary use of the tested material:** | Healing patch for superficial dermal wounds, medical device | |

Two extracts of the tested material in polar and non-polar solvent were tested to assess the potential for skin sensitization using murine local lymph node assay. The study is part of medical device biocompatibility testing.

The local lymph node assay (LLNA) with incorporation of 3H-methylthymidine radionuclide was used. The testing was performed according to the standard CSN EN ISO 10993-10: Biologick6 hodnocení zdravotnických prostředků - Část 10: Zkoušky dráždivosti a senzibilizace kǔže, adopted on 01 March 2014 (Czech version of the European standard EN ISO 10993-10: Biological evaluation of medical devices - Part 10: Tests for skin sensitization). In this study, the contact allergenic potential of the material was evaluated after local application to female BALB/c mice. Mice were applied 100% polar extract of the tested material and 100% non-polar extract of the tested material for 3 consecutive days.

### Polar extract of the tested material

In the LLNA test, the animals were exposed to the polar extract of the tested material, this polar extract of the tested material did not induce any sensitization response. The cell proliferation results were negative, indicating that the polar extract of the tested material is not a contact allergen for female mice. The polar extract of the tested material gives a negative sensitization response in the LLNA test.

### Non-polar extract of the tested material

In the LLNA test, the animals were exposed to the non-polar extract of the tested material, this non-polar extract of the tested material did not induce any sensitization response. The cell proliferation results were negative, indicating that the non-polar extract of the tested material is not a contact allergen for female mice. The non-polar extract of the tested material gives a negative sensitization response in the LLNA test.

The basic principle of the LLNA method is that sensitizers induce primary proliferation of lymphocytes in the lymph nodes draining instead of application of tested material. The rate of proliferation is proportional to the applied dose (and allergen potential) and provides a simple means of obtaining an objective quantitative measurement of sensitization. The LLNA test evaluates proliferation in the groups of tested extracts (polar and non-polar solvent) by comparison with proliferation in the control groups with the solvent (separately for each solvent - polar and non-polar), called the Stimulation Index (SI). This index serves to evaluate the sensitization potential of the tested material. The method is based on the use of radioactive labeling to measure cell proliferation.

The extracts were prepared using polar (Aqua pro injectione) and non-polar solvent (olivae oleum raffinatum).

For three consecutive days, an open application of prepared extracts of the tested material (polar or non-polar) was performed on the dorsal part of each ear. At the same time, the application of the solvents themselves (polar or non-polar) took place as a control.

At the end of the experiment, the incorporation of 3H-methylthymidine into the tissue of the auricular lymph nodes was measured with a scintillation apparatus.

The stimulation index (for 3H-methylthymidine incorporation) was calculated from the values of the exposed and control groups.

Verification of study acceptability criteria is performed periodically in our laboratory using the positive control 1chloro-2,4-dinitrobenzene. The experiment was performed simultaneously with this study and the results are a part of the primary data of the study.

**Table 13: Classification of animals into groups**

| | **Group 1 Solvent (polar)** | **Group 2 Polar extract of the tested material** | **Group 3 Solvent (non-polar)** | **Group 4 Non-polar extract of the tested material** |
|---|---|---|---|---|
| Number of animals | 5 | 5 | 5 | 5 |
| Animal No. | 1-5 | 6-10 | 11-15 | 16-20 |
| Hreeding vessel No. | 1 | 2 | 3 | 4 |

| | | | | |
|---|---|---|---|---|
| Dose volume: 25 µl / ear / animal Frequency of preparation: Each day of application | | | | |

### Application

For three consecutive days, an open application of 25 µl (in the morning hours, using a pipette) of prepared extracts of the tested material (polar or non-polar), solvents (polar or non-polar), was performed on the dorsal part of each ear.

Carboxymethyl cellulose (0.5% w/v) was used for better adhesion of the polar solvent and the extract in the polar solvent at the place of application.

### Test schedule

Day 1:
An open application of 25 µl to the dorsal part of each ear (in the morning hours, using a pipette) of prepared extracts of the tested material (polar or non-polar), solvents (polar or non-polar), or positive controls were performed.

Day 2 and 3:
Repetition of the application procedure performed on Day 1.

Day 4 and 5:
No application.

Day 6:
250 µl of phosphate buffer (PBS) containing 3.06 MBq/ml of 3H-methylthymidine was injected intravenously into the tail vein of all animals. Five hours later, all the animals were killed.

Day 7:
Incorporation of 3H-methylthymidine into the tissue of auricular lymph nodes was measured with a scintillation apparatus.

### Examination in vivo - Mortality

During the test, the health status of the animals was monitored, most often at the time when the animals were handled - during applications twice a day.

### Clinical observation

Clinical observation was performed according to the internal regulation SOP M/1, chapter 4.1 - during applications twice a day. All changes in the behavior and health of the animals were recorded, such as piloerection, lacrimation, appearance of skin, fur and mucous membranes, ataxia, tremors and convulsions, aggressiveness, change in grooming, significant change in activity and change in frequency and intensity of breathing, such as shortness of breath and others. The onset and duration of observed symptoms were monitored and characterized. During clinical observations, an examination of skin irritation at the place of application was performed.

### Body weight

The body weight of the animals was recorded using an electronic scale. Weighing was performed on the first day before the application and on the day of the autopsy before the application of the radionuclide.

### Autopsy

On the sixth day (five hours after the application of the radionuclide), all experimental animals were killed by prolonged anesthesia.

### Post-mortem examination

Immediately after the planned killing of the animals, both ears were cut off and a diameter of 8mm (= 0.5 cm²) ear target was cut from the apical part of each ear using a disposable punch. The ear targets were weighed on analytical scales.

### Incorporation of 3H-methylthymidine

The tissue of both lymph nodes was transferred to 1 ml of PBS (phosphate buffer) using a fine mechanical de-aggregation through a 100µm nylon sieve. A pair of lymph nodes was analyzed separately for each animal in the group. Cells were washed twice with excess PBS and then precipitated with 5% trichloroacetic acid (TCA) at 4 °C for 18 hours. The sediments were resuspended in 1 ml of TCA and transferred to scintillation vials containing 10 ml of scintillation fluid. Incorporation of 3H-methylthymidine was measured using a β-scintillation apparatus (Beckmann LS 6500) in disintegrations per minute (DPM).

### 1/ Polar extract of the tested material

In the group of animals applied with a polar solvent, no changes were recorded. During the experiment, the animals exposed to the polar extract of the test material showed no skin reaction and no clinical signs of intoxication. Body weight and body weight gain were not significantly changed.

The mean value of DPM in the group of animals applied with the polar extract of the tested material was slightly reduced compared to the group of animals applied with the polar solvent. The SI value for animals in the polar extract group of the tested material is lower than the threshold level (stimulation index SI is <3).

The weight of the ear targets in the animals applied with the polar extract of the tested material and in the group of animals applied with the polar extract was relatively balanced.

A comparison of the stimulation indices and DPM values between the group of animals applied with the extract of the test substance in a polar solvent and the group of animals applied with a polar solvent showed that the tested material did not cause a significant increase in radioisotope incorporation into the DNA of dividing lymphocytes, so the result of the LLNA test is negative. SI in all groups was <3.

### 2/ Non-polar extract of the tested material

The extract of the tested material in a non-polar solvent caused only a very slight erythema on the skin of the ears and restlessness in the animals. The same reaction was recorded also in animals applied with the non-polar solvent alone.

Body weight gain and body weight in animals applied with the non-polar extract of the tested material as well as in animals applied with the non-polar solvent alone was slightly lower, but not statistically significantly.

The DPM value in animals applied with the non-polar extract of the tested material was practically the same compared to the animals applied with the non-polar solvent alone. The SI value for both groups was below the threshold level (stimulation index SI is <3).

The weight of the ear targets in animals applied with the non-polar extract of the tested material and in the group of animals applied with the non-polar extract was relatively balanced.

A comparison of the stimulation indices and DPM values between both groups (extract with non-polar solvent and non-polar solvent alone) showed that the tested material did not cause any significant increase in radioisotope incorporation into the DNA of dividing lymphocytes, so the result of the LLNA test is negative. SI in all groups was <3.

### Example 4

The biomass produced by the method according to Example 1 was applied to a nanotechnological substrate in the form of a film. Biomass treated in this way was used to support the healing of hypertrophic scars and keloids.

Film with biomass was applied on an open bleeding wound on the injured person's hand so that it covered the entire wound. The injured person felt numbness in the wound within a few minutes and the bleeding from the wound stopped. After 2 hours, the wound was still calm, without signs of inflammation or bleeding. After 24 hours from applying the film with biomass, the film was removed from the wound. The wound was sealed, the formed scar was free of inflammation and it was subsequently healing without any problems.

### Example 5

The biomass produced by the method according to Example 1 was applied to a nanotechnological substrate in the form of a film. Biomass treated in this way was used to fully cover a wound caused by a burn, it was a 2^{nd} degree burn, which was accompanied by the loss of the epidermis with imminent damage to the dermis and the subsequent formation of necrosis and scabs. The injured person felt numbness in the wound within a few minutes after the application of the biomass with the film. After 2 hours from the application of the film with biomass, the wound was still calm, without signs of inflammation and numbed. A healing process followed without inflammation and without the formation of necrosis, which was accompanied also by a decrease in swelling and blister fluid within 24 hours after application.

After 24 hours, the wound was treated by applying a new film with biomass. A big advantage is that the film does not stick to the wound and after absorption of the active substance, it separates by itself.

### Example 6

The biomass produced by the method according to Example 1 was applied to a nanotechnological substrate in the form of a film. Biomass treated in this way was used to fully cover inflamed skin at the site of acne. The skin affected by acne was festering. The skin remained intact; the film was applied to the inflamed part. The treated person felt numbness in the area of skin inflammation within a few minutes. After 2 hours from the application of the film with biomass, the affected skin was soothed, without signs of inflammation, and still numbed. After 24 hours from the application of the film with biomass, the film was removed from the inflamed acneic skin. The skin was sealed and without inflammation, and was healing without problems.

### Example 7

The biomass produced by the method according to Example 1 was applied to a nanotechnological substrate in the form of a film. The tests with the name of the study "BIOKOŽA Zkouška lokálních účinků po implantaci do podkožní tkáně" were performed by the Výzkumný ústav organických syntéz a.s., testing device SLP - Centrum ekologie, toxikologie a analytiky, No. 296, 533 54 Rybitví, Czech Republic.

### Number of study: 23V02322/34

Beginning and end of the experimental phase of the study: 31. 05. - 20. 09. 2023. The goal of the study was to evaluate the local effects of the tested material after implantation into the subcutaneous tissue of rats. The study was executed according to the CSN EN ISO 10993-6 methodology.

The study evaluated the biological reactions of the tested material after implantation into the subcutaneous tissue. This study is a part of the evaluation of the biocompatibility of medical materials.

Conclusion of the study: The tested material did not cause any pathological reaction at the implantation site. Local tissue changes in response to the test material were designated as "no or minimal response" based on the overall assessment score.

The present invention is subject to constant and ongoing testing, which will continue well into the future.

### Industrial applicability

The method of preparation of biomass to support healing of hypertrophic scars and keloids and biomass produced in this way has a wide range of applications in the field of medicine as well as in the field of skin healing research. Biomass produced in this way will allow faster and more hygienic closure of wounds, especially those with problematic healing, while ensuring rapid absorption of inflammation as well as numbing of the affected skin.

## Claims

1. The method of preparation of biomass to support healing of hypertrophic scars and keloids, **characterized in that** it contains the steps:
Culturing of mycelium of the filamentous fungus,
Preparation of a pure culture of the filamentous fungus,
Verification of the generic and species affiliation of the filamentous fungus,
and Finalization of the biomass;
whereby, when finalizing the biomass, a spore inoculum is prepared from a settled culture of filamentous fungus grown on PDA growth medium in 0.01% Tween water, then the culture is flooded over the entire surface with Tween water and the conidia are mechanically released, the mixture of mycelia and conidia is then filtered through sterile gauze, then the number of conidia 10⁸ /ml is determined in a Bürker chamber and the conidia are then used to inoculate the growth medium in at least one culture vessel, and after 48 hours of culturing on a shaker 250 rpm, at 27 °C, mycelium is separated from the growth medium by filtration under reduced pressure, subsequently mycelium is captured on filter paper, then it is dried at 50°C for 1 hour, and the dried biomass is crushed to a fine powder, which is then placed in a freezer at a temperature of -20°C.

2. The method of preparation of biomass according to claim 1 **characterized in that,** when cultivating the mycelium of a filamentous fungus, the growth media are first sterilized in an autoclave at 120°C and 120 kPa for 20 minutes, then the mycelium is transferred from the stock mixture to inoculate 100 ml of SGB, and subsequently the mycelium is cultured dynamically for 7 days on a shaker 250 rpm at 27°C.

3. The method of preparation of biomass according to claim 1 **characterized in that,** when preparing a pure culture of a filamentous fungus, the cultured mycelium is transferred to solidified SGA and PDA media, where it is incubated for 3 days, subsequently for 5 days identical colonies of filamentous fungi are formed and conidia are formed on the colonies, which are subsequently transferred to fresh PDA and SGA growth media.

4. The method of preparation of biomass according to claim 1 **characterized in that** the genus and species affiliation of the filamentous fungus is verified after 5 days of culturing, microscopically.

5. The method of preparation of biomass according to claims 1-4 **characterized in that** the biomass is crushed into a fine powder by application of liquid nitrogen.

6. The method of preparation of biomass according to claims 1-5 **characterized in that** as a filter medium to trap mycelium, blue paper filters are used.

7. The biomass produced by the method according to claims 1-6, which is applied to a nanotechnological substrate in the form of a film.

8. The use of biomass according to claim 7 to support healing of hypertrophic scars and keloids or to test medical substances intended for the skin.
